# EUROPEAN PATENT SPECIFICATION

(11) **EP 3 620 138 B1**
(45) Date of publication and mention of the grant of the patent: **02.08.2023**
(21) Application number: 19195842.0
(22) Date of filing: 06.09.2019
(51) Int. Cl.: A61F 5/455

(54) **MENSTRUAL CUP**
MENSTRUATIONSBECHER
COUPELLE MENSTRUELLE

(30) Priority: 07.09.2018 US 201816124262
(43) Date of publication of application: 11.03.2020
(73) Proprietor: The Procter & Gamble Company, Cincinnati, OH 45202 (US)
(72) Inventor: STOEBE-LATHAM, Rebecca, Cincinnati, Ohio 45202 (US)
(74) Representative: P&G Patent Germany

(56) References cited:
- WO-A1-2006/058409
- WO-A1-2007/082341
- US-A1- 2017 049 609
- Rosey Reusables: "JuJu Cup Model 4 - Menstrual Cup Review", YouTube, 20 February 2018 (2018-02-20), pages 1-3, XP054980135, Retrieved from the Internet: URL:https://www.youtube.com/watch?v=is3Hla xN-64 [retrieved on 2020-01-22]
- Rosey Reusables: "JuJu Cup Model 4 vs. Super Jennie Small (Menstrual Cup Comparison)", YouTube, 1 February 2018 (2018-02-01), pages 1-1, XP054980134, Retrieved from the Internet: URL:https://www.youtube.com/watch?v=6gIcY2 Gf7qM [retrieved on 2020-01-22]
- Anonymous: "Choosing the right size menstrual cup. What model JuJu cup do I need?", , 22 January 2020 (2020-01-22), XP055660809, Retrieved from the Internet: URL:https://www.juju.com.au/menstrual-cup- size-guide/ [retrieved on 2020-01-22]
- "JuJu & Organicup Menstrual Cup Comparison", Youtube, 2 April 2014 (2014-04-02), page 3 pp., XP054980149, Retrieved from the Internet: URL:https://www.youtube.com/watch?v=zjBbtq A2ZN0 [retrieved on 2020-01-24]

## Description

### FIELD OF THE INVENTION

The present disclosure generally relates to a menstrual cup having specific geometric properties to optimize comfort and protection. The disclosure further relates to an array of packages comprising menstrual cups or different sizes and to a kit comprising a menstrual cup and an absorbent article or disposable wipe.

### BACKGROUND OF THE INVENTION

Menstrual cups, and other vaginal discharge collection devices are well known. The first menstrual cups were designed in 1930 but have only become more popular commercially over the past 10 years. While user acceptance of such devices is increasing, there are still many issues with existing menstrual cups. Menstrual cups are intended to be re-used, requiring changing and cleaning once every 8 to 12 hours, dependent on flow. Menstrual cups also need to be comfortable, they need to maintain their position within a user's body and they need to be easy to insert and remove. This results in multiple natural tradeoffs when designing menstrual cups.

For example, if the shape of a menstrual cup is rounder, it will generally be easier to insert and remove, but may push more on the bladder resulting in discomfort for a user. Alternatively, cups that are very stiff tend to open and seal to vaginal walls easily, but stiff cups can conform to the body less and may be less comfortable or may shift more during use. Thus, there exists some natural tradeoffs between, for example, capacity of the cup and comfort or flexibility of the cup and rigour.

Cups that are currently on the market have differing design choices that lead to shortcomings in the product design. For example, one cup has a very round base, which increases overall capacity of the cup, but that pushes against the bladder and may cause difficulty with urination. Another available cup is long and formed of more flexible material, which can cause discomfort and may not open as easily as other cups. There are various such problems with cups present on the market today.

Thus, there exists a need for a cup that solves one or more of the above identified problems.

WO 2007/082341 A1 discloses a vaginal cup provided with increased radial rigidity.

### SUMMARY OF THE INVENTION

The present invention relates to a menstrual cup according to claim 1.

### BRIEF DESCRIPTION OF THE DRAWINGS

FIG. 1 is a perspective view of one example of a menstrual cup as described herein.
FIG. 2 is a front view of the menstrual cup in an upright position, indicating the vertical and horizontal axes.
FIGs. 3A and 3B show different dimensions of the cup when viewed through a cross-section.
FIG. 4 shows dimensions of the wall of the menstrual cup when viewed through cross-section x-x.

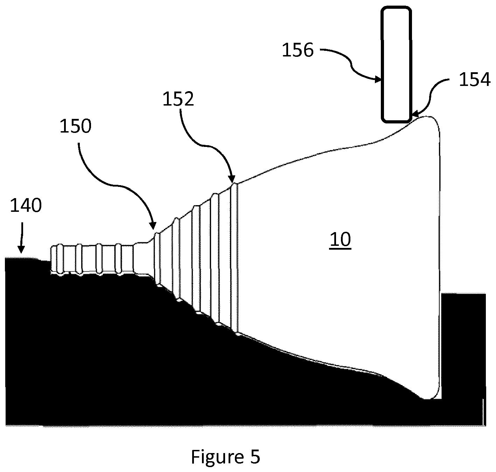

FIG. 5 schematically illustrates the process of taking measurements according to the compression test method.
FIGs. 6 and 7 show in graph format the relative fit of prior art menstrual cups.
FIG. 8 shows in graph format the relative fit of the menstrual cup described herein.

While the specification concludes with claims particularly pointing out and distinctly claiming the subject matter that is regarded as the present invention, it is believed that the invention will be more fully understood from the following description taken in conjunction with the accompanying drawings. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. The drawings are not necessarily to scale.

### DETAILED DESCRIPTION

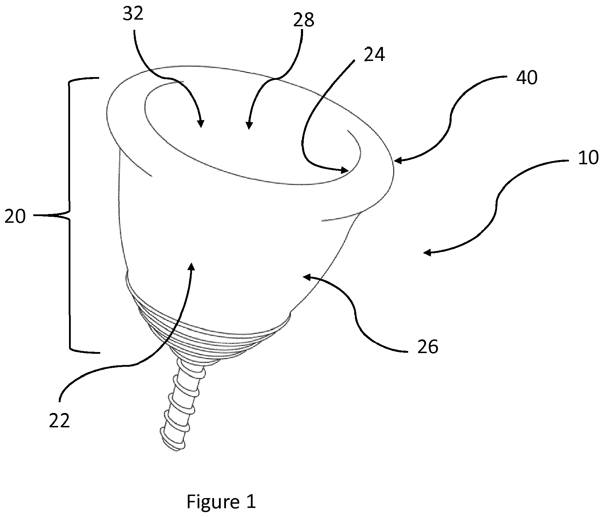

Reference is made herein to a menstrual cup 10, as shown in Figure 1. However, it will be appreciated that the device described may be used for collection of other vaginal fluids. To insert the menstrual cup in a user's vagina, an open end of the cup must first be folded in on itself and held in folded position by a user. Once located in position, the user releases the cup, at which point it unfolds and nestles within the user's vagina. Thus, the cup needs to be sufficiently flexible to enable folding ready for insertion, but it should also have enough resilience to resume its original position once released. To remain in position, the cup must have a certain width such that it remains "wedged" around the cervical OS, without being so wide that it causes discomfort for a user. It is known that when menstrual cups are too big, they may cause discomfort and/or e.g., press on the bladder of a user. To remove the cup, a user grasps the base of the cup or, where applicable, a stem provided at the base of the cup and slowly pulls the cup from the vagina. During this process, the open end of the cup squeezes together, temporarily reducing the capacity of the cup. If there is too much liquid within the cup during removal, there may be some spillage. Conversely, for optimal usage time without having to replace and/or clean the cup, the cup should have a certain capacity in line with the flow of average users. Thus, there are many trade-offs that must be made and factors that should be balanced.

Furthermore, for optimal usage, it may be necessary to provide an array of cups that cater for different needs, e.g. vagina size and fluid flow. The present inventors have discovered that the needs of most users can be met with appropriately chosen dimensions of two or more cups as described herein.

### Receptacle

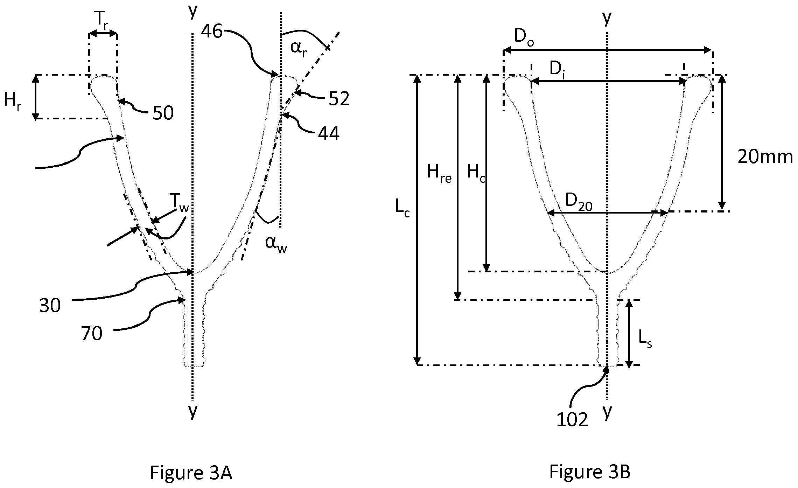

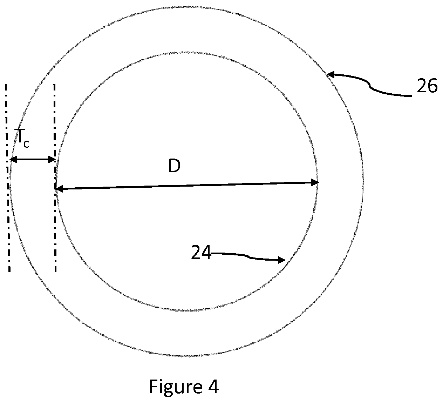

The cup 10, which is adapted for use in a vagina, includes a receptacle 20 having a wall 22 with an inner wall surface 24 which defines a cavity 32 adapted for collecting fluid and an opposed outer wall surface 26. The receptacle extends from an open top end 28 to a closed bottom end 30 (shown in Figure 3A). When viewed through the y-y cross-section (as shown in Figures 3A & 3B), the receptacle has a generally "V" shaped cross-section. When viewed through the x-x cross-section (as shown in Figure 4), the receptacle has a generally cylindrical cross-section that increases in diameter from the base of the receptacle to the open top end.

The wall includes an upper rim portion 40 which strengthens the top end of the receptacle, maintaining the cup in a pre-selected position when in situ and providing appropriate resiliency for the cup to resume its shape once in situ. The wall further includes a main portion 42 extending from the bottom end towards the top end. The wall additionally includes a transition portion 44 between the main portion and the rim portion.

The thickness of the cup wall, measured between the inner wall surface 24 and the outer wall surface 26 varies from the rim portion 40 to the main portion 42, as best seen in Figure 3A & 3B. Generally, the thickness of the wall decreases from the rim portion 40 to the transition portion 44 and from the transition portion 44 to the main portion 42. The rim portion of the wall is rounded at its top end 46 and forms the thickest part of the wall, thus strengthening the top end and maintaining both cup shape and position when in situ. The transition portion 44 of the wall tapers from the thicker rim portion to the main portion which is narrower. The transition portion permits the cup to maintain a substantially smooth profile for increased comfort during use due.

Preferably, the thickness Tᵣ of the rim portion of the wall, as measured at the thickest part of the rim in a direction substantially orthogonal to the vertical axis of the cup, is between 4 mm and 5 mm, 4.25 mm and 4.75 mm measured between the inner surface 50 and the outer surface 52 of the rim. A ratio of Tᵣ to T_{w} is preferably at least 1.1:1, 1.2:1, 1.25:1, 1.3:1, 1.4:1 or 1.5:1.

The rim further has a height Hᵣ measured in a direction substantially parallel to the longitudinal axis from the open top end of the receptacle to the transition portion, where the thickness of the wall reduces. Preferably, the height Hᵣ is between 5 mm and 15 mm, 6 mm and 13 mm, 7 mm and 11 mm or 8mm and 9mm.

The outer surface 52 of the rim portion of the wall further has an angle αᵣ relative to the longitudinal axis that is greater than the angle α_{w} of the outer surface of the main portion of wall relative to the longitudinal axis. The point at which the angle changes is also at the transition portion 44 of the wall. Without being bound by theory, it is thought that this increased angle provides better anchorage for the cup when in situ. Preferably, the angle of the outer surface of the upper rim portion relative to the longitudinal axis is between 28° and 34°, 29° and 33°, 30° and 32° or 31°.

The rim preferably has a rounded profile to improve comfort of the cup when in situ.

The thickness of the wall T_{w} remains generally constant through the main portion, although it will be appreciated that this thickness could vary, for example, becoming narrower towards the open top end (to the rim). The narrower wall thickness in the main portion allows ready folding of the cup for both insertion into and removal from the vagina. Preferably, the main portion of the wall has a thickness T_{w} of between 2 mm and 4 mm, 2.5 mm and 3.5 mm or 3 mm.

At the transition portion, there is preferably a radius of curvature between the wall at the rim and the wall at the main portion of between 6 mm and 9 mm or 7 mm and 8 mm. The radius of curvature ensures a smooth transition from the rim to the main portion of the wall, thereby ensuring a comfortable fit while the cup is in situ and smooth entry and removal of the cup from the user's vagina.

As the dimensions described above (wall thickness at different points, angle of outer surface of rim relative to longitudinal axis and height of rim) are chosen for their properties in terms of shape maintenance, foldability and resilience, it is anticipated that they may be kept the same throughout an array of cups of different sizes. Thus, preferably, the first and second cups of an array of cups have substantially the same wall thickness (Tᵣ and T_{w}), Rim angle (αᵣ), rim height (Hᵣ) and radius of curvature.

As mentioned briefly above, the receptacle has a general "V-shaped" cross-section. Compared with existing menstrual cups, it has been found that the "V-shaped" cross-section provides an optimal shape that balances the need for a wide open end to hold the cup in place when in situ, with a narrower lower end to avoid discomfort e.g., caused by the cup pushing on the bladder, while maximising the overall capacity of the cup.

Considering first the outer surface of the wall, the receptacle has a diameter Dₒ measured at the open top end measured between the widest points of the cup at the upper rim portion of the wall in a direction substantially orthogonal to the longitudinal axis. A second diameter D₂₀ is measured between the outer surface of the wall at a point 20 mm away from the open top end of the receptacle (in a direction parallel substantially parallel to the longitudinal axis). The relationship between these diameters provides an indication of the overall structure of the cup. Preferably, the ratio of D₂₀ to Dₒ should be less than 0.9, preferably less than 0.85, preferably less than 0.8.

Preferably Dₒ is between 40 mm and 55 mm, 42 mm and 53 mm, 45 mm and 49 mm, 46 mm and 48 mm, 46 mm and 54 mm, 47 mm and 53 mm or 52 mm and 54 mm. In an array of cups of different sizes, preferably a first cup has a diameter Do of between 45 mm and 49 mm, 46 mm and 48 mm or 47 mm and a second cup has a diameter Do of between 51 mm and 55 mm, 52 mm and 54 mm or 53 mm. It is anticipated that this range of rim diameters in an array of cups would fit the 86 percentile of potential users.

The receptacle further has a height Hᵣₑ measured from the open top end of the receptacle to an external base 70 in a direction substantially parallel to the longitudinal axis. Preferably, the height Hᵣₑ is between 43 mm and 50 mm, 44 mm and 49 mm, 45 mm and 48 mm, 46 mm and 47 mm. Where different sizes of cups are provided, a first cup may have a height Hᵣₑ of between 43 mm and 49 mm, 44 mm and 48 mm, 45 mm and 47 mm or 46 mm and the second cup may have a height Hᵣₑ or between 46 mm and 51 mm, 47 mm and 50 mm, 48 mm and 49 mm.

The capacity of the cup is determined by the inner dimensions of the receptacle, for example, the inner diameter, Dᵢ, measured at the open top end between inner surfaces of the wall, and the cavity depth H_{c} measured from the open top end to the cavity base. Furthermore, the capacity of the cup varies when in use from a "resting position" i.e., when in situ in a user's vagina or when outside of the body or in "compressed" position, i.e., during insertion or removal from the user's vagina. The difference between the compressed capacity and the resting capacity should not be so extreme that any liquids held in the cup are spilled during removal of the cup. Thus, in embodiments, the receptacle or cup has a resting capacity Cᵣ of between 20 mL and 40 mL, 22 mL and 38 mL. As described above, where an array of cups is provided, preferably a first cup has a capacity of between 20 mL and 28 mL, 21 mL and 27 mL, 22 mL and 26 mL, 23 mL and 25 mL or 24 mL and a second cup has a capacity of between 33 mL and 41 mL, 34 mL and 40 mL, 35 mL and 39 mL, 36 mL and 38 mL or 37 mL.

The depth of the cavity as measured between the open top end and the cavity base in a direction substantially parallel to the longitudinal axis is between between 40 mm and 47 mm, 41 mm and 46 mm, 42 mm and 45 mm, 43 mm and 44 mm. Where different sizes of cups are provided, a first cup may have a cavity depth H_{c} of between 40 mm and 46 mm, 41 mm and 45 mm, 42 mm and 44 mm or 43 mm and the second cup may have a cavity height H_{c} of between 46 mm and 51 mm, 47 mm and 50 mm, 48 mm and 49 mm.

The diameter of the cavity D; as measured between the inner surfaces of the wall at the open top end may be between 36 mm and 46 mm, 38 mm and 44 mm, 40 mm and 42 mm. In an array of cups of different sizes, preferably a first cup has a diameter Dᵢ of between 36 mm and 40 mm and a second cup has a diameter Dᵢ of between 42 mm and 46 mm.

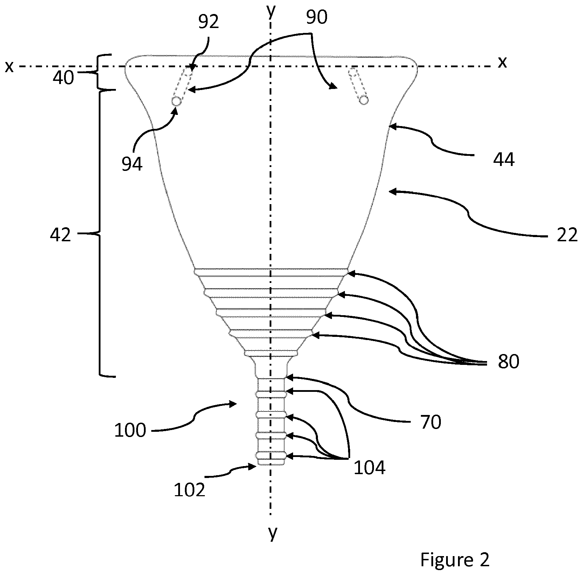

Preferably, the receptacle further comprises one or more holes 90 (shown in Figure 2) extending from the inner wall surface to the outer wall surface within the upper rim portion of the wall. The holes are preferably radially spaced apart from each other. When the cup is substantially upright, as shown in Figure 2, the inner end 92 of each hole is positioned higher than the outer end 94 of the hole relative to the longitudinal axis. The holes permit fluid communication between the inner wall surface and the outer wall surface such that as the cup is inserted into the vagina and removed therefrom, air pressure within the cavity is equalised with atmospheric pressure. The inclined angle of the holes limits the chance that fluid that has accumulated within the cavity leaks out through the holes, either when the cup is in situ or as it is removed. Preferably, the cup has between 2 and 8 holes, preferably between 3 and 5 holes or 4 holes. Preferably, the holes have a diameter of between 0.5 mm and 2.5 mm or 1 mm and 2 mm.

The receptacle further comprises grips 80 provided on the outer surface of the receptacle adjacent the base. The grips 80 shown in Figure 2 are in the form of concentric circles, however it will be appreciated that the grips may take other forms, for example, the grips may be in the form of concentric zigzags, curves, parallel vertical or horizontal lines or a series of concentric dots. The grips are preferably provided from the external base of the receptacle up to a distance that is 40%, 35%, 30% or 25% of the receptacle height. In an embodiment, the receptacle is provided with 2, 3, 4, 5 or 6 grips, spaced between 1 mm and 5mm, 2 mm and 4 mm or 3 mm and 3.5 mm apart and protruding a thickness of between 0.5 mm and 1.25 mm, 0.5 mm and 1.1 mm from the outer surface of the receptacle (measured in a direction orthogonal to a tangent of the outer surface). Without being bound by theory, it has been found that placement of the grips can have a serious impact on both ease of use and comfort when in situ. Grips are preferred by consumers to enable easy removal of the device when in situ, however, if grips are position too high on the outer surface of the receptacle wall or if they protrude too far from the receptacle outer surface, they may cause unnecessary discomfort to the user.

### Stem

A stem 12 is provided to aid extraction of the cup once in situ. The stem extends from the base of the receptacle and is substantially co-axial with the central axis of the receptacle. As shown in Figures 2 and 3B, the stem is generally elongate relative to the receptacle and has a substantially rectangular cross-section with a rounded base 102. The stem may have a uniform cross-section along its length or it may vary in thickness. Preferably, the stem has a cross-sectional area of between 5.5 mm² and 7.5 mm², 5.75 mm² and 7.25 mm², 6 mm² and 7 mm², 6.25 mm² and 6.75 mm² or 6.5 mm². The stem further has a length Lₛ measured from the external base of the receptacle to the base of the stem of between 13 mm and 32 mm, 15 mm and 30 mm, 17.5 mm and 27.5 mm, 20 mm and 25 mm. The stem should be sufficiently long to enable a user to firmly grasp it for removal of the cup, however, the stem should not be so long as to cause discomfort to the user when in situ. To enable cups having overall same or similar lengths, the size of the stem may be varied. For example, in an array having a normal sized cup (the first cup) and a larger cup (the second cup), the first cup may have a longer stem than the second cup. For example, the first cup may have a stem of between 18 mm and 20 mm in length, whereas the second cup may have a stem of between 12 mm and 14 mm in length.

The stem may be hollow or solid. Preferably, the stem is solid such that it can easily be snapped at different points by the user, thus providing flexibility for the user to choose a length they find practical and comfortable. The stem is further provided with grips 104 in the form of concentric rings. It will be appreciated that the grips may take other forms, for example, zig zags or vertical struts and that any number of grips may be provided. Preferably, the stem has between 3 and 7 grips, spaced between 1mm to 4 mm or 2 mm to 3 mm apart and protruding between 0.5 mm and 1.2 mm or 0.6 mm and 1.0 mm from the body of the stem.

The total length of the menstrual cup, including the stem and receptacle measured from the top open end (thus including rim) to the bottom of the stem 102 preferably does not exceed 70mm, 69 mm, 68 mm, 67 mm, 66 mm or 65 mm.

The above dimensions are all chosen to balance the tension between the outward size and comfort of the cup, the flexibility of the cup and finally, the capacity of the cup. The capacity of the cup varies when in use and can be measured in "resting" position, i.e., when in situ in a user's vagina or when outside of the body or in "compressed" position, i.e., during insertion or removal from the user's vagina.

### Array of Cups

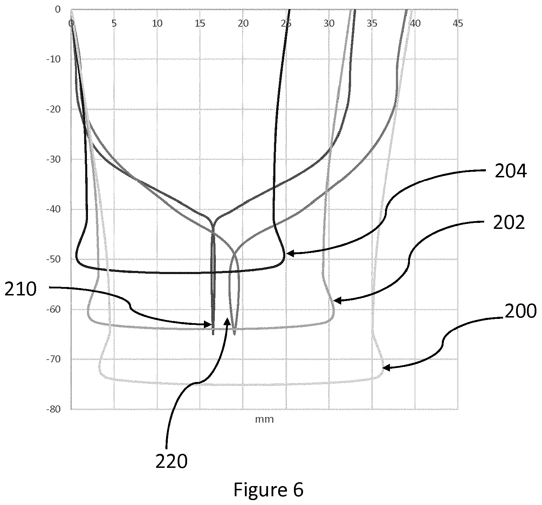

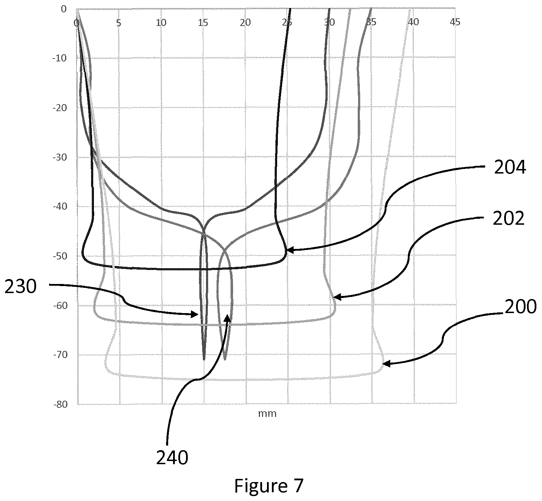

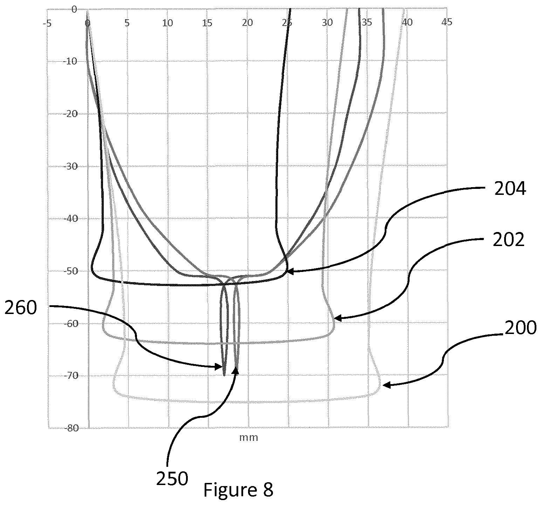

As described above, different cups may be provided having different dimensions to meet the needs of different users. The needs may be based on body size/dimensions or blood flow during menstruation. To date, there are no "one size fits all" cups available and, as a result, most commercially available cups are only suitable for a very small percentage of the female population. Some manufacturers have started making cups in two different sizes - typically a "regular" and "large". However, the present inventors have found that cups currently available on the market still do not meet the needs of most consumers and are typically still considered to be uncomfortable by many females. The dimensions of the leading menstrual cups in the market are shown in Tables I (regular size) and II (large size) below and Figures 6 and 7 show how this relates to average vagina sizes. Average vagina width at the cervical opening is 32.5, with a standard deviation of 7.13, however, width varies with age. Because the ruggae of the vagina can be stretched to accommodate a range of widths, larger width cups will nearly always offer a better stay-in-place experience than smaller cups. However, larger cups are also more likely to put pressure on the bladder or rectum. The benefit of the "V" shaped cup described herein is also illustrated in Figure 8, where it can be seen that the present cup(s) meet the needs of consumers by having a wide and thick rim but avoid pressing on the bladder or rectum. Without being bound by theory, it is thought that the cups described herein are suitable for use for approximately the 86% percentile of women and, based on conservative estimates, should be useful for between 11 hours to 19 hours of fluid flow (compared with an average tampon user).

**TABLE I**

| **Regular** | Comparative Cup 1 | Comparative Cup 2 | Inventive Cup (N) |
|---|---|---|---|
| L_{c} (mm) | 70 | 71 | 65 |
| Dₒ (mm) | 44 | 41 | 47 |
| Dᵢ (mm) | 34 | 30 | 38 |
| D₂₀ (mm) | - | - | 26 |
| Capacity (mL) | 27 | 23 | 24 |
| H_{c} (mm) | 53 | 45 | 44 |
| T_{w} (mm) | 3 | 2.5 | 3 |
| Tᵣ (mm) | 3.5 | 2.5 | 4.5 |

**TABLE II**

| **Large** | Comparative Cup 1 | Comparative Cup 2 | Inventive Cup (L) |
|---|---|---|---|
| L_{c} (mm) | 70 | 71 | 65 |
| Dₒ (mm) | 48 | 45 | 53 |
| Dᵢ (mm) | 37 | 35 | 44 |
| D₂₀ (mm) | - | - | 32 |
| Capacity (mL) | 33 | 35 | 37 |
| H_{c} (mm) | 53 | 49 | 49 |
| T_{w} (mm) | 3 | 2.5 | 3 |
| Tᵣ (mm) | 3.5 | 2.5 | 4.5, |

Figures 6, 7 and 8 show different vagina sizes as follows: average 200, 25^{th} percentile 202 and 5^{th} percentile 204. Comparative Cup 1 Regular 210 and Large 220 are shown in Figure 6, Comparative Cup 2 Regular 230 and Large 240 are shown in Figure 7, Inventive Cup Regular 250 and Large 260 are shown in Figure 8.

The above described "normal" and "large" menstrual cups may be displayed as an "array" of packages comprising menstrual cups, or other like-branded products, having different sizes as described above. While two different sizes of menstrual cups are described herein, it will be appreciated that such an array may comprise more than two different sizes, or even styles or materials, of cups. Said packages may have the same brand and/or sub-brand and be oriented in proximity to each other within a given retail store (or advertised online together). An array is marketed as a line-up of products normally having like packaging elements (e.g., packaging material type, film, paper, dominant colour, design theme, etc.) that conveys to consumers that the different individual packages are part of a larger line-up. Arrays often have the same brand, for example, "Tampax", and same sub-brand, for example "Tampax Pearl". A different array may have the brand "Tampax" and the sub-brand "Tampax Naturals".

The packages may feature the same overall look, but use different colour combinations to indicate differences (e.g., capacity or functionality) to users. In the present example, an array of menstrual products may comprise one or more menstrual cups that share the same brand identifier and one or more disposable absorbent products, for example: tampons, sanitary or incontinence pads or wipes that share the same brand identifier. Alternatively, the branding of complimentary products may be different, but they may be sold together as a kit of products. For example, such a kit may comprise one or more menstrual cups of different sizes branded as "Tampax" and liners for use with the menstrual cups branded as "Always" or wet wipes with other branding. Such kits will likely be packaged together to clearly indicate to consumers that they should be used in conjunction with one another.

### Materials

The cup may be formed of an elastomeric material, such as an organosilicone oxide polymer, i.e., a silicone rubber. Silicone rubber is preferred because it rarely (if ever) causes skin irritation, and it has the necessary resiliency and durability. Furthermore, silicone rubber may be placed in boiling water for sterilization without damaging the article. Preferably, the silicone rubber is of medical grade.

The receptacle and stem are preferably formed integrally and of the same material. Although it will be appreciated that it would be possible to form the parts separately and then to attach them by known means.

The cup may be formed by an injection molding process. However, it will be appreciated that other known methods of forming articles from plastics and other similar materials may be used. Where the cup is formed by a molding process, preferably the holes are introduced during the molding process (as compared with boring holes through the rim wall after molding). By forming the holes as part of the molding process, this increases the ease of cleaning the cup.

### Compression of Cup

The dimensions of rim and wall thickness and the materials making up the cup influence how the cup behaves upon entry and exit of a user's vagina and while in situ. The present inventors have found that the dimensions and materials chosen above provide optimal conditions for this and, in particular, enable the cup to stay in place while being comfortable before, during and after use. The compression test method below measures the force required to flex the cup (as described below). Preferably, a cup as described herein requires a compression force of between 1N - 5N, 1.5N to 4.5N or 2.0N to 4.0N at the top (as defined below) of the cup, a compression force of between 4.0N to 8.5N, 4.5N to 8.0N or 5.0N to 7.5N at the middle of the cup and a compression force of between 4.5N to 13.5N, 5.0N to 12.0N, 5.5N to 11:0N, 6.0N to 10.0N, 6.5N to 9.0N or 7.0N to 8.0N at the bottom.

The following results are the average forces measured using the compression test method on a series of 210 cups having the dimensions of Inventive Cups N and L, shown in Tables II and III:

**Table III:**

| | | Mean Load (N) | Range (N) |
|---|---|---|---|
| Inventive Cup (N) | Top | 3.17 | 2.7-3.8 |
| | Middle | 6.49 | 5.3-7.6 |
| | Bottom | 7.97 | 5.8-7.1 |
| Inventive Cup (L) | Top | 2.65 | 2.2-3 |
| | Middle | 5.79 | 5-6.6 |
| | Bottom | 7.44 | 2.8-12.1 |

### Compression Test for Menstrual Cup

The maximum compression strength at three different location on a menstrual cup is measured on a constant rate of extension tensile tester with compression load cell capability. A suitable instrument is the MTS Alliance using Testworks 4.0 Software, as available from MTS Systems Corp., Eden Prairie, MN, or equivalent, equipped with a load cell for which the forces measured are within 10% to 90% of the limit of the cell. All testing is performed in a room controlled at 23°C ± 2 C° and 50% ± 2% relative humidity and test samples are conditioned in this environment for at least 2 hours prior to testing.

The bottom stationary fixture consists of a rigid, horizontal platen large enough to support the sample holder, described herein. The upper fixture is a cylindrical plunger having an overall length of about 30 mm with a diameter of 6.0 mm. The contact tip is a ball nose having a radius of 3.0 mm. The plunger has an adapter compatible with the mount on the load cell capable of securing the plunger orthogonal to the bottom platen.

The sample holder is constructed in such a way that the cup is supported with its radial axis (depicted as y in Figure 2) oriented parallel to the bottom horizontal plate. The sample holder must be rigid, nondeformable and the interior must conform to the shape of the cup's surface. The holder is deep enough to allow one half of the cup (divided along the radial axis, depicted as y) to rest securely inside the holder and it enables testing of the cup at different locations parallel to its radial axis. One suitable way to make said sample holder is to create a 3D-printed plastic mold. This type of design ensures the test sample remains in a fixed position during testing while also allowing the re-positioning necessary to analyze multiple locations.

For the purpose of this test, the longitudinal end of the test sample that is open to collect fluid and may contain a rim will be known as the "top" (region 40 in Figure 2). The longitudinal end of the test sample that is at the base of the receptacle (excluding any stem present) will be known as the "bottom" (region 70 in Figure 2).

Obtain five substantially similar test samples. Mark the test locations (measured parallel to the radial axis) on the outer surface of the test sample as follows. The Bottom Location is 5.0 mm from the bottom of the cup receptacle. The Mid Location is above the bottom of the cup receptacle at a distance that is equal to 30.0% of the distance between the top and bottom of the cup receptacle determined along the radial axis. The Top Location is 5.0 mm from the top edge of the cup receptacle.

Program the tensile tester as a compression test to lower the crosshead at 2.0 mm per second. The ball-nose plunger moves down until the desired displacement is reached. Displacements for each test location are as follows. Bottom Location is 3.0 mm, Mid Location is 5.0 mm and Top Location is 10.0 mm. Force (N) and displacement (mm) are recorded at a data acquisition rate of 50 Hz. Insert the test sample into its respective holder and place it onto the bottom platen of the tensile tester. Align the pre-marked test location under the center of the ball nose of the plunger. At low speed, lower the crosshead until it exerts a negligible force (< 0.03 N) onto the test sample at the test location, then zero the crosshead. Start the test, ensuring the correct displacement is used for the respective test location. Record the test location and the Maximum Peak Force to the nearest 0.01 N.

Repeat in like fashion for all 3 test locations on all 5 replicates. Calculate the arithmetic mean for Maximum Peak Force to the nearest 0.01 N for each test location (Bottom, Mid, Top) and report as Compression Strength for each test location.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A menstrual cup (10) comprising a receptacle (20) having an open top end (28) and a closed base, the receptacle comprising:
a. a rim (40) extending around the circumference of the top end and have an inner diameter Dᵢ across the top end of more than 35mm and a thickness Tᵣ of between 3 mm and 5 mm; and
b. a length of less than 53 mm from the top end to the base of the receptacle,
wherein the receptacle has a wall (22) extending from a base of the rim to the base of the receptacle, the wall having a thickness T_{w} of between 2 mm and 4.5 mm.

2. A menstrual cup as claimed in claim 1, wherein the rim (40) has a depth of between 7mm to 10 mm measured from the top end of the receptacle (20) towards the base.

3. A menstrual cup as claimed in claim 1 or claim 2, wherein the cup has a diameter D₂₀ measured a distance of 20 mm from the top end in a direction parallel to the inner diameter, wherein a ratio of D₂₀ to Dᵢ is less than 0.9.

4. A menstrual cup as claimed in any one of claims 1-3, wherein the ratio of T_{w} to Tᵣ is less than 0.8.

5. A menstrual cup as claimed in any preceding claim, wherein the transition (44) from rim to receptacle wall is a concave curve with radius of at between 6 mm and 9 mm.

6. A menstrual cup as claimed in any preceding claim, wherein the rim (40) requires a compression force of at least 1N as measured by the "Compression Test for Menstrual Cup" test method defined herein.

7. A menstrual cup as claimed in any preceding claim, having a resting capacity of between 20 mL and 40 mL.

8. A menstrual cup as claimed in any preceding claim, wherein the receptacle (20) has one or more grips (80) provided on an external base of the receptacle.

9. A menstrual cup as claimed in claim 8, wherein the one or more grips (80) are provided between the external base and a distance that is 40% of the receptacle height.

10. A menstrual cup as claimed in claims 8 or 9, wherein the grips (80) are in the form of concentric circles protruding a distance of between 0.5 mm and 1.25 mm from an external wall of the receptacle.

11. A menstrual cup as claimed in any preceding claim, further comprising a stem extending from the external base of the receptacle.

12. A menstrual cup as claimed in claim 11, wherein the stem has a cross-sectional area between 5.5 mm² and 7.5 mm² and a length of between 15 mm and 30 mm.

13. A menstrual cup as claimed in claims 11 or 12, wherein the combined length of the receptacle and stem is less than 70 mm.

14. A menstrual cup as claimed in any preceding claim, wherein the cup is formed as an integral piece and is formed of organosilicone oxide polymer.

## Patentansprüche

1. Menstruationstasse (10), umfassend einen Behälter (20), der ein oben offenes Ende (28) und einen geschlossenen Boden aufweist, der Behälter umfassend:
a. einen Rand (40), der sich um den Umfang des oberen Endes erstreckt und einen Innendurchmesser Dᵢ über das obere Ende hinweg von mehr als 35 mm und eine Dicke Tᵣ zwischen 3 mm und 5 mm aufweist; und
b. eine Länge von weniger als 53 mm von dem oberen Ende zu dem Boden des Behälters,
wobei der Behälter eine Wand (22) aufweist, die sich von einem Boden des Randes zu dem Boden des Behälters erstreckt, wobei die Wand eine Dicke T_{w} zwischen 2 mm und 4,5 mm aufweist.

2. Menstruationstasse nach Anspruch 1, wobei der Rand (40) eine Tiefe zwischen 7 mm und 10 mm aufweist, gemessen von dem oberen Ende des Behälters (20) zu dem Boden hin.

3. Menstruationstasse nach Anspruch 1 oder 2, wobei die Tasse einen Durchmesser D₂₀ aufweist, gemessen in einem Abstand von 20 mm von dem oberen Ende in einer Richtung parallel zu dem Innendurchmesser, wobei ein Verhältnis von D₂₀ zu Dᵢ weniger als 0,9 beträgt.

4. Menstruationstasse nach einem der Ansprüche 1 bis 3, wobei das Verhältnis von T_{w} zu Tᵣ weniger als 0,8 beträgt.

5. Menstruationstasse nach einem der vorstehenden Ansprüche, wobei der Übergang (44) von Rand zu Behälterwand eine konkave Kurve mit einem Radius von zwischen 6 mm und 9 mm ist.

6. Menstruationstasse nach einem der vorstehenden Ansprüche, wobei der Rand (40) eine Kompressionskraft von mindestens 1N erfordert, gemessen durch das hierin definierte Prüfverfahren "Kompressionsprüfung für Menstruationstassen".

7. Menstruationstasse nach einem der vorstehenden Ansprüche, die ein Ruhevolumen zwischen 20 mL und 40 mL aufweist.

8. Menstruationstasse nach einem der vorstehenden Ansprüche, wobei der Behälter (20) einen oder mehrere Griffe (80) aufweist, die an einem Außenboden des Behälters bereitgestellt sind.

9. Menstruationstasse nach Anspruch 8, wobei der eine oder die mehreren Griffe (80) zwischen dem Außenboden und einem Abstand, der 40 % der Behälterhöhe beträgt, bereitgestellt sind.

10. Menstruationstasse nach Anspruch 8 oder 9, wobei die Griffe (80) in der Form von konzentrischen Kreisen vorliegen, die in einem Abstand zwischen 0,5 mm und 1,25 mm von einer Außenwand des Behälters vorstehen.

11. Menstruationstasse nach einem der vorstehenden Ansprüche, ferner umfassend einen Stamm, der sich von dem Außenboden des Behälters erstreckt.

12. Menstruationstasse nach Anspruch 11, wobei der Stamm eine Querschnittsfläche zwischen 5,5 mm² und 7,5 mm² und eine Länge zwischen 15 mm und 30 mm aufweist.

13. Menstruationstasse nach Anspruch 11 oder 12, wobei die kombinierte Länge des Behälters und des Stamms weniger als 70 mm beträgt.

14. Menstruationstasse nach einem der vorstehenden Ansprüche, wobei die Tasse als ein integrales Stück ausgebildet ist und aus Organosiliciumoxidpolymer gebildet ist.

## Revendications

1. Coupelle menstruelle (10) comprenant un réceptacle (20) ayant une extrémité supérieure ouverte (28) et une base fermée, le réceptacle comprenant :
a. un rebord (40) s'étendant autour de la circonférence de l'extrémité supérieure et ont un diamètre interne Dᵢ à travers l'extrémité supérieure de plus de 35 mm et une épaisseur Tᵣ comprise entre 3 mm et 5 mm ; et
b. une longueur inférieure à 53 mm de l'extrémité supérieure à la base du réceptacle,
dans laquelle le réceptacle a une paroi (22) s'étendant d'une base du rebord à la base du réceptacle, la paroi ayant une épaisseur T_{w} comprise entre 2 mm et 4,5 mm.

2. Coupelle menstruelle selon la revendication 1, dans laquelle le rebord (40) a une profondeur comprise entre 7 mm et 10 mm mesurée de l'extrémité supérieure du réceptacle (20) vers la base.

3. Coupelle menstruelle selon la revendication 1 ou la revendication 2, dans laquelle la coupelle a un diamètre D₂₀ mesuré une distance de 20 mm de l'extrémité supérieure dans une direction parallèle au diamètre interne, dans laquelle un rapport de D₂₀ sur Dᵢ est inférieur à 0,9.

4. Coupelle menstruelle selon l'une quelconque des revendications 1 à 3, dans laquelle le rapport de T_{w} sur Tᵣ est inférieur à 0,8.

5. Coupelle menstruelle selon l'une quelconque revendication précédente, dans laquelle la transition (44) de rebord à paroi de réceptacle est une courbe concave avec un rayon compris entre 6 mm et 9 mm.

6. Coupelle menstruelle selon l'une quelconque revendication précédente, dans laquelle le rebord (40) nécessite une force de compression d'au moins 1 N telle que mesurée par le procédé d'essai « Essai de compression pour coupelle menstruelle » défini ici.

7. Coupelle menstruelle selon l'une quelconque revendication précédente, ayant une capacité au repos comprise entre 20 ml et 40 ml.

8. Coupelle menstruelle selon l'une quelconque revendication précédente, dans laquelle le réceptacle (20) a un ou plusieurs dispositifs de préhension (80) pourvus sur une base externe du réceptacle.

9. Coupelle menstruelle selon la revendication 8, dans laquelle les un ou plusieurs dispositifs de préhension (80) sont pourvus entre la base externe et une distance qui est de 40 % de la hauteur de réceptacle.

10. Coupelle menstruelle selon les revendications 8 ou 9, dans laquelle les dispositifs de préhension (80) sont sous la forme de cercles concentriques faisant saillie sur une distance comprise entre 0,5 mm et 1,25 mm d'une paroi externe du réceptacle.

11. Coupelle menstruelle selon l'une quelconque revendication précédente, comprenant en outre une tige s'étendant de la base externe du réceptacle.

12. Coupelle menstruelle selon la revendication 11, dans laquelle la tige a une aire en coupe transversale comprise entre 5,5 mm² et 7,5 mm² et une longueur comprise entre 15 mm et 30 mm.

13. Coupelle menstruelle selon les revendications 11 ou 12, dans laquelle la longueur combinée du réceptacle et de la tige est inférieure à 70 mm.

14. Coupelle menstruelle selon l'une quelconque revendication précédente, dans laquelle la coupelle est formée sous forme d'une pièce solidaire et est formée de polymère d'oxyde d'organosilicone.
